# EUROPÄISCHE PATENTSCHRIFT

(11) **EP 3 256 173 B1**
(45) Veröffentlichungstag und Bekanntmachung des Hinweises auf die Patenterteilung: **09.05.2018**
(21) Anmeldenummer: 16708093.6
(22) Anmeldetag: 18.02.2016
(51) Int. Cl.: A61L 2/20, B01B 1/00

(54) **BLITZVERDAMPFERANORDNUNG MIT BLITZVERDAMPFER SOWIE BETRIEBSVERFAHREN**
RAPID EVAPORATOR ARRANGEMENT WITH RAPID EVAPORATOR, AND OPERATING METHOD
SYSTÈME D'ÉVAPORATION ÉCLAIR COMPRENANT UN ÉVAPORATEUR ÉCLAIR, ET PROCÉDÉ DE FONCTIONNEMENT

(30) Priorität: 20.02.2015 DE 102015102491
(43) Veröffentlichungstag der Anmeldung: 20.12.2017
(73) Patentinhaber: Metall + Plastic GmbH, 78315 Radolfzell (DE)
(72) Erfinder: VON STENGLIN, Christoph, 78315 Radolfzell (DE)
(74) Vertreter: Patentanwälte Behrmann Wagner PartG mbB
(86) Internationale Anmeldenummer: PCT/EP2016/053482
(87) Internationale Veröffentlichungsnummer: WO 2016/131931

(56) Entgegenhaltungen:
- EP-A2- 1 193 462
- WO-A1-2006/108796
- WO-A1-2011/076400

## Beschreibung

Die Erfindung betrifft eine Vorrichtung (Blitzdampferzeuger) zum Erzeugen von Dekontaminationsmitteldampf, insbesondere Wasserstoffperoxiddampf gemäß dem Oberbegriff des Anspruchs 1, umfassend einen ein- oder mehrteiligen Verdampferkörper, eine Heizeinrichtung zum Erhitzen des Verdampferkörpers sowie mindestens einen Zuführkanal, bevorzugt mehrere Zuführkanäle zum Zuführen von zu verdampfender Dekontaminationsmittelflüssigkeit, insbesondere Wasserstoffperoxid, zu mindestens einem von mehreren in dem Verdampferkörper angeordneten Sacklöchern und einem oberhalb von oberen Sacklochrändern der Sacklöcher angeordneten und einen Trägermedium-Einlass mit einem Auslass gasleitend verbindenden Strömungskanal für ein gasförmiges Trägermedium, insbesondere Luft, zum Abführen des Dekontaminationsmitteldampfes in einer Strömungsrichtung des Trägermediums durch den Auslass.

Ferner betrifft die Erfindung eine, vorzugsweise pharmazietechnische, Anordnung, umfassend einen zu dekontaminierenden Raum, insbesondere einen Isolator und/oder eine Schleuse sowie eine Vorrichtung zur Erzeugung von Dekontaminationsmitteldampf gemäß Anspruch 10.

Darüber hinaus betrifft die Erfindung ein Verfahren zum Betreiben einer Vorrichtung zum Erzeugen von Dekontaminationsmitteldampf.

Zur Dekontamination von Isolatoren und/oder Schleusen wird in der Pharmaindustrie aufgrund seiner hohen Reaktivität Wasserstoffperoxyddampf eingesetzt. Dieser wird durch Verdampfen einer wässrigen Wasserstoffperoxydlösung erhalten. Zur Minimierung einer Explosionsgefahr bei der Verdampfung von wasserstoffperoxydhaltigen Lösungen sind sogenannte Blitzverdampfer (Blitzdampferzeuger) im Einsatz, mit dem Ziel, kontinuierlich kleine Mengen von wasserstoffperoxydhaltiger Flüssigkeit schlagartig (blitzartig) zu verdampfen. Ein Sieden größerer Mengen an wasserstoffperoxydhaltiger Flüssigkeit ist aufgrund der vorgenannten Explosionsgefahr nicht zulässig. Die Schwierigkeit beim Verdampfen kleiner Mengen wasserstoffperoxydhaltiger Flüssigkeit, insbesondere von wässrigen Lösungen ist die Bildung von auf heißer Verdampferfläche "tanzender" Flüssigkeitstropfen, die den Bestrebungen einer blitzartigen Verdampfung zuwiderlaufen.

Aus der DE 10 2006 006 095 A1 ist ein Wasserstoffperoxyddampferzeuger bekannt, der eine ebene Verdampferfläche aufweist. Hier kann es zu der vorerwähnten "tanzenden" Tröpfchenbildung kommen.

Aus der EP 0 972 159 B1 ist ein alternativer Blitzverdampfer (Schnellverdampfer) bekannt, der sich durch hydraulisch miteinander kommunizierend angeordnete Verdampferkanäle in einem Verdampferkörper auszeichnet. Der Aufbau ist vergleichsweise komplex.

Hinsichtlich weiteren Standes der Technik wird auf die DE 602 03 603 T2 oder die DE 603 00 820 T2 verwiesen.

Aus der DE 2005 030 822 A1 ist ein Wasserstoffperoxydverdampfer mit einem topfartigen Gehäuse und einem Verdampferkörper bekannt, der eine einzige, großflächige Verdampferoberfläche aufweist, wobei die Wärmezufuhr in das Dekontaminationsmittel ausschließlich von unten erfolgt. Der bekannte Verdampfer scheint hinsichtlich seiner Verdampfungsrate sowie hinsichtlich der Vermeidung einer "tanzenden" Dekontaminationsmitteldampftröpfchen verbesserungsbedürftig. Aus der DE 2005 030 822 A1 ist es zudem bekannt, mehrere Verdampfer über jeweils eine Leitung an ein zu sterilisierendes Gefäß anzuschließen, um die Dekontaminationsdampfmenge zu erhöhen. Die gesamten Verdampferkosten fallen also x-Mal an. Zudem müssen eine Vielzahl von Dampfleitungen in den zu verdampfenden Raum geführt werden, was bei kleinen Räumen aus Platzgründen problematisch ist. Zudem müssen dann eine Vielzahl von Dichtungen vorgesehen werden.

Aus der CN 2009 43844 Y ist ein Verdampfer für Wasser bekannt. Der bekannte Verdampfer weist einen Verdampfungskörper mit einer Vielzahl kleiner Löcher auf. Diesen ist gemeinsam ein einziger Zulaufkanal zugeordnet, der mittig oberhalb des Verdampferkörpers angeordnet ist. Damit die Vielzahl von kleinen Löchern ihren Beitrag zur Verdampfung leisten können, muss durch den einzigen Zuführkanal eine ausreichend große Flüssigkeitsmenge zugeführt werden, was dann jedoch dem Ziel einer spontanen Blitzverdampfung von Dekontaminationsmitteln zuwider laufen würde. In der Praxis käme es zu einem gefährlichen Verkochen von Dekontaminationsmitteln. Daher ist der bekannte Verdampfer nicht zum Verdampfen von Dekontaminationsmitteln geeignet.

Aus der EP 1 738 777 A1 ist ein Verdampfer für eine Sterilisationsappartur mit vier Zuführkanälen bekannt, durch die eine Dekontaminationsmittelflüssigkeit auf eine beheizbare Platte gesprüht wird. Die Verdampferoberfläche kann Vertiefungen beispielsweise in der Form von Halbkugeln aufweisen.

Sämtliche vorgenannten Blitzverdampfer zeichnen sich durch einen vergleichsweise komplexen Aufbau und/oder durch eine verbesserungswürdige Verdampfungsrate aus.

Von der Anmelderin wurde in der EP 2 448 602 B1 ein gegenüber dem vorgenannten Stand der Technik deutlich verbesserter Blitzverdampfer vorgeschlagen, der sich dadurch auszeichnet, dass in dem ein- oder mehrteiligen Verdampferkörper des Blitzverdampfers mehrere Sacklöcher vorgesehen sind, denen jeweils mindestens einer der Zuführkanäle zugeordnet ist und wobei die Zuführkanäle derart angeordnet sind, dass das zu verdampfende Dekontaminationsmittel unmittelbar in die Sacklöcher eintropfbar ist. Dieser verbesserte Blitzverdampfer zeichnet sich durch ein hohe Verdampfungsrate aus und es werden "tanzende" Dekontaminationsmittelflüssigkeitstropfen innerhalb des Verdampfers weitgehend vermieden, da die Verdampfung innerhalb von umfangsgeschlossenen Sacklöchern im Verdampferkörper stattfindet. Der verbesserte Blitzverdampfer hat sich bewährt - es bestehen jedoch Bestrebungen die Auswahlsicherheit zu erhöhen, insbesondere für den Fall, dass einer der Zuführkanäle versagen sollte.

Aus der EP 1 193 462 A2, bei dem es sich um einen fachfremden Stand der Technik handelt, ist ein Wärmetauscher zum Verdampfen von Treibstoff für eine Verbrennungsanlage bekannt, bei dem ein Strömungskanal unterhalb von Durchgangsöffnungen zum Einleiten von Kraftstoff angeordnet ist.

Ausgehend von dem vorgenannten Stand der Technik liegt der Erfindung daher die Aufgabe zugrunde, einen Blitzverdampfer für die Dekontaminationsmittel anzugeben, der sich durch eine hohe Betriebssicherheit auszeichnet, bei gleichzeitiger hoher Verdampfungsrade unter zumindest weitgehender Vermeidung "tanzender" Dekontaminationsmitteltropfen.

Ferner besteht die Aufgabe darin, eine (Dekontaminations-)Anordnung mit einem zu dekontaminierenden Raum und einem entsprechend verbesserten Blitzverdampfer anzugeben sowie ein optimiertes Betriebsverfahren für einen erfindungsgemäßen Blitzverdampfer.

Diese Aufgabe wird hinsichtlich des Blitzverdampfers mit den Merkmalen des Anspruchs 1 gelöst, d.h. bei einem gattungsgemäßen Blitzverdampfer dadurch, dass mindestens zwei der Sacklöcher, bevorzugt sämtliche Sacklöcher, mit Abstand zu ihrem jeweiligen oberen Sacklochrand flüssigkeitsleitend miteinander verbunden sind.

Hinsichtlich der Anordnung wird die Aufgabe mit den Merkmalen des Anspruchs 10 gelöst und hinsichtlich des Betriebsverfahrens mit den Merkmalen des Anspruchs 11, d.h. bei einem gattungsgemäßen Verfahren dadurch, dass in eines der Sacklöcher zugeführte Dekontaminationsmittelflüssigkeit über die flüssigkeitsleitende Verbindung in eines der Sacklöcher fließt und dort zu Dekontaminationsmitteldampf verdampft wird.

Vorteilhafte Weiterbildungen der Erfindung sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Zur Vermeidung von Wiederholungen sollen vorrichtungsgemäß offenbarte Merkmale als verfahrensgemäß offenbart gelten und beanspruchbar sein. Ebenso sollen verfahrensgemäß offenbarte Merkmale als vorrichtungsgemäß offenbart gelten und beanspruchbar sein.

Der Erfindung liegt der Gedanke zugrunde, mindestens zwei in dem Verdampferkörper angeordnete Sacklöcher mit Abstand zu den Sacklochoberseiten, d.h. unterhalb eines umfangsgeschlossenen Abschnitts jedes Sackloches flüssigkeitsleitend miteinander zu verbinden, bevorzugt durch miteinander verbundene, so dass die in eines der Sacklöcher zugeführte Dekontaminationsmittelflüssigkeit mit Abstand zu dem oberhalb der Sacklöcher ausgebildeten Strömungskanal in mindestens ein benachbartes Sackloch strömen kann. Anders ausgedrückt ist beabstandet zu dem Strömungskanal, in dem der in den Sacklöchern entstehende und nach oben aufsteigende Dekontaminationsmitteldampf mit Hilfe eines Trägermediums, insbesondere Luft, in den zu dekontaminierenden Raum abtransportiert wird, mindestens einen Verbindungskanal zwischen zwei Sacklöchern im Verdampfungskörper anzuordnen, der eine Dekontaminationsmittelflüssigkeitsverteilung zwischen mindestens zwei Sacklöchern ermöglicht. Hierdurch kann die Verdampferkapazität eines Sackloches, bei dem die Dekontaminationsmittelflüssigkeit, beispielsweise aufgrund eines verstopften Zuführkanals unterbrochen ist weiter genutzt werden, um aus mindestens einem anderen der Sacklöcher nachströmende Dekontaminationsmittelflüssigkeit zu verdampfen und damit eine kontinuierliche Versorgung des zu dekontaminierenden Raums mit einem hohen Dekontaminationsmitteldampfvolumenstrom sicherzustellen. So garantiert der erfindungsgemäße Blitzverdampfer (Schnellverdampfer) aufgrund des Vorsehens der Sacklöcher im Verdampferkörper eine hohe Verdampfungsrate und ist zudem durch eine erhöhte Ausfallsicherheit gekennzeichnet, da selbst für den Ausfall einer oder mehrerer Zuführleitungen eine große Verdampferfläche zur Verfügung steht. Bei einem nach dem Konzept der Erfindung ausgebildeten Schnellverdampfer kann der zu verdampfenden, vorzugsweise tropfenförmig zugeführten Dekontaminationsmittelflüssigkeit in kürzester Zeit eine große Wärmemenge zugeführt werden, und zwar nicht nur von unten, sondern auch durch Strahlungswärme von den Umfangswänden der Sacklöcher her. Bevorzugt handelt es sich bei der zu verdampfenden Dekontaminationsmittelflüssigkeit um eine wässrige Lösung von Wasserstoffperoxid, ganz besonders bevorzugt um eine 35%ige bis 50%ige Lösung.

Besonders bevorzugt ist eine Ausführungsform, bei der den Sacklöchern jeweils mindestens einer der Zuführkanäle zugeordnet ist und die Zuführkanäle derart angeordnet sind, dass die zu verdampfende Dekontaminationsmittelflüssigkeit unmittelbar in jedes der Sacklöcher zuführbar, insbesondere eintropfbar ist. Die erfindungsgemäße Ausgestaltung des Blitzverdampfers ermöglicht jedoch auch eine alternative, konstruktiv einfachere und kostengünstigere Ausführungsvariante, bei der bewusst auf einzelne Zuführkanäle verzichtet wird, d.h. bei der nicht sämtlichen Sacklöchern sondern zumindest einem der flüssigkeitsleitend miteinander verbundenen Sacklöcher ein Zuführkanal zugeordnet ist und die Verteilung der Dekontaminationsmittelflüssigkeit nicht oder nicht ausschließlich über die Zuführkanäle erfolgt, sondern zumindest zum Teil über die flüssigkeitsleitenden Verbindungen unterhalb des Strömungskanals. Auch bei einer solchen Ausführungsform werden aufgrund der Verteilung der Dekontaminationsmittelflüssigkeit "tanzende" Tropfen vermieden und es wird die hohe Verdampfungskapazität der Sacklöcher genutzt.

Besonders zweckmäßig ist es, unabhängig von der Wahl einer der zuvor erläuterten Ausführungsvarianten, wenn die vorhandenen Zuführkanäle, bevorzugt senkrecht zur Längserstreckung des Strömungskanals, durch den Strömungskanal hindurchgeführt sind, beispielsweise in der Form von Injektionsnadeln, und unterhalb des jeweiligen oberen Sacklochrandes innerhalb des jeweiligen Sackloches münden, bevorzugt derart, dass die aus den Zuführkanälen austretende Dekontaminationsmittelflüssigkeit bei senkrechtem Fall nach unten direkt auf den Sacklochgrund tropfen kann. Durch das Durchdringen bzw. Durchsetzen des Strömungskanals mit dem mindestens einen Zuführkanal kann ein Mitreißen der Dekontaminationsmittelflüssigkeitstropfen mit dem Trägermedium in Richtung Auslass sicher verhindert werden.

Im Hinblick auf die konkrete Ausgestaltung der Sacklöcher gibt es unterschiedliche Möglichkeiten. Bevorzugt ist es, wenn diese eine größere Tiefen- als Breitenerstreckung aufweisen. Anders ausgedrückt, ist der maximale Durchmesser der Sacklöcher in einem umfangsgeschlossenen Bereich, d.h. oberhalb einer flüssigkeitsleitenden Verbindung größer als die jeweilige Tiefenerstreckung. Als besonders vorteilhaft hat sich eine zylindrische Konturierung des umfangsgeschlossenen Bereichs oberhalb der flüssigkeitsleitenden Verbindung herausgestellt.

Wie erwähnt zeichnen sich die Sacklöcher durch einen Abschnitt mit umfangsgeschlossener Mantelfläche aus, welcher an den oberen Sacklochrand angrenzt. Unterhalb dieses umfangsgeschlossenen Mantelflächenbereichs ist dann die erfindungsgemäß flüssigkeitsleitende Verbindung vorgesehen.
Bevorzugt sind die Sacklöcher in einem Vollmaterialblock (Verdampferblock) angeordnet und noch weiter bevorzugt als Bohrungen hergestellt, wodurch ein äußerst einfacher und effektiver Aufbau der Verdampfungsvorrichtung gewährleistet wird.

Besonders zweckmäßig ist es, die Heizeinrichtung derart auszubilden und/oder anzusteuern, dass diese den Verdampferkörper zumindest im Bereich der Sacklöcher auf eine Temperatur aus einem Temperaturbereich zwischen 100°C und etwa 140°C erhitzt. Ganz besonders bevorzugt beträgt die Temperatur im Verdampferbetrieb etwa 120°C oder weniger, um die nicht schlagartig verdampfenden Tropfen auf der Verdampferoberfläche, d.h. am Sacklochgrund optimal zu verhindern.

Besonders bevorzugt ist eine Ausführungsform des Blitzverdampfers, bei der die flüssigkeitsleitende Verbindung zwischen mindestens zwei Sacklöchern derart ausgebildet ist, dass diese die tiefsten Bereiche, d.h. die Sacklochgründe bzw. Sacklochböden mit einer geraden (ebenen, bevorzugt horizontalen) Verbindungsebene verbindet - anders ausgedrückt befinden sich die tiefsten Bereiche der flüssigkeitsleitend miteinander verbundenen Sacklöcher sowie der Boden bzw. Grund der Verbindung, insbesondere eines Verbindungskanals in einer gemeinsamen Ebene, um eine gleichmäßige Verteilung der Dekontaminationsmittelflüssigkeit zu gewährleisten und um ein Aufstauen in der flüssigkeitsleitenden Verbindung bzw. in dem Verbindungskanal zu verhindern. Durch die wie beschrieben gestaltete flüssigkeitsleitende Verbindung wird zudem gewährleistet, dass Flüssigkeit keine Stufe nach oben überwinden muss, um in den Bereich der flüssigkeitsleitenden Verbindung bzw. den Verbindungskanal zu gelangen.

Wie bereits angedeutet, ist die flüssigkeitsleitende Verbindung zwischen mindestens zwei Sacklöchern bevorzugt als umfangsgeschlossener, mit Abstand zum Strömungskanal angeordneter Kanal im Verdampferkörper ausgebildet. Besonders bevorzugt ist es, wenn zumindest einer der Sacklöcher über jeweils einen solchen Verbindungskanal mit einem weiteren bzw. benachbarten Sackloch flüssigkeitsleitend verbunden ist. Der Verbindungskanal überwindet dabei den Abstand zwischen zwei benachbarten Sacklöchern in der Art eines Tunnels unterhalb des Strömungskanals.

Anstelle der Realisierung einzelner Verbindungskanäle zwischen benachbarten Sacklöchern ist es alternativ auch denkbar, einen gemeinsamen Verbindungsraum zu schaffen, der bevorzugt nicht durch in diesem angeordnete senkrechte bzw. vertikale Säulen unterbrochen ist - hierdurch kann die unmittelbar beheizte Bodenfläche und damit die effektive Verdampferfläche noch weiter vergrößert werden.

Unabhängig von der konkreten Ausgestaltung der mindestens einen flüssigkeitsleitenden Verbindung als Verbindungskanal oder gemeinsamer, insbesondere nicht in der Flächenerstreckung, beispielsweis durch eine in Hochrichtung verlaufende Säule unterbrochener Raum, zeichnet sich die flüssigkeitsleitende Verbindung dadurch aus, dass diese nach oben hin von einem Deckenbereich begrenzt ist der unterhalb des Strömungskanals angeordnet ist - anders ausgedrückt besteht in einer flüssigkeitsleitenden Verbindung kein unmittelbar senkrechter Verbindungsweg zum Strömungskanal - vielmehr muss der Dekontaminationsmitteldampf erst seitlich und dann in den Sacklöchern nach oben in den Strömungskanal aufsteigen.

Als besonders zweckmäßig hat es sich herausgestellt, wenn eine Grund- bzw. Bodenfläche der flüssigkeitsleitenden Verbindung, insbesondere eines Verbindungskanals als mit der Heizeinrichtung beheizbare Verdampferfläche ausgebildet ist, so dass bereits auf dieser Dekontaminationsmitteldampf erzeugt werden kann, wobei der Dekontaminationsmitteldampf über die jeweils zu dem Verbindungskanal benachbarten bzw. über den Verbindungskanal miteinander verbundenen Sacklöcher nach oben aufsteigen kann. Durch diese Maßnahme wird die Verdampfungsleistung bei gleichbleibender Baugröße weiter erhöht. Die Verdampfungsleistung innerhalb des flüssigkeitsleitenden Bereichs ist aufgrund der Begrenzung dieses Bereichs durch eine Deckenfläche besonders hoch.

Als besonders zweckmäßig hat es sich herausgestellt, wenn die flüssigkeitsleitende Verbindung, insbesondere ein vorerwähnter Verbindungskanal oder der gemeinsame Verbindungsraum aus dem Vollen gefertigt ist, d.h. durch Materialabtrag innerhalb eines Materialblocks, insbesondere eines Edelstahlblocks, insbesondere durch Fräsen in radialer Richtung bezogen auf die Längsmittelachse der Sacklöcher. Dies kann beispielsweise dadurch realisiert werden, dass eine Fräseinrichtung in ein Sackloch eingeführt und dann seitlich, d.h. in radialer Richtung hin zum benachbarten Sackloch verstellt wird.

Durch die Ausbildung der flüssigkeitsleitenden Verbindung in einem Vollmaterialkörper werden Dichtigkeitsprobleme, wie diese auftreten könnten, wenn der Verdampferkörper im Bereich der flüssigkeitsleitenden Verbindung schalenartig bzw. zweigeteilt ausgebildet wird, sicher verhindert werden. Wesentlich ist also, dass der Verdampferkörper zumindest im Bereich der flüssigkeitsleitenden Verbindung nicht mehrteilig ausgebildet ist bzw. keine Stoß- oder Verbindungsstelle aufweist, sondern als Vollmaterialblock ausgestaltet ist.

Wie erläutert, ist der obere Rand der Sacklöcher senkrecht zur Strömungsrichtung des Trägermediums im Strömungskanal zwischen Einlass und Auslass über einen Vollmaterialbereich beabstandet, in welchem die Sacklöcher umfangsgeschlossen ausgestaltet sind. Im Hinblick auf die geometrische Ausgestaltung des Strömungskanals gibt es nun unterschiedliche Möglichkeiten. Gemäß einer ersten Alternative weist der Strömungskanal einen ebenen Boden auf, so dass die Sacklochränder der mit Abstand zu einem Boden flüssigkeitsleitend miteinander verbundenen Sacklöcher, bevorzugt sämtlicher Sachlöcher in einer gemeinsamen Ebene angeordnet sind. Alternativ und bevorzugt ist eine Ausführungsform realisierbar, bei der die oberen Sacklöcher nicht in einer gemeinsamen Ebene angeordnet sind, sondern in einem gekrümmten Bodenbereich des Strömungskanals. Diese Ausführungsform ermöglicht eine gekrümmte, insbesondere zylindrische Ausgestaltung des Strömungskanals, was zu optimierteren Strömungsverhältnissen in dem Kanal führt.

Auch im Hinblick auf die relative Anordnung zweier flüssigkeitsleitend miteinander verbundener Sacklöcher zueinander gibt es unterschiedliche alternativ, bevorzugt jedoch kumulativ realisierbare Möglichkeiten. So können mindestens zwei flüssigkeitsleitend miteinander verbundene Sacklöcher in Richtung der Strömungsrichtung des Trägermediums im Strömungskanal voneinander beabstandet sein und/oder senkrecht zu dieser Strömungsrichtung. Besonders bevorzugt ist zumindest eine Viereranordnung von Sacklöchern, bei der die Längsmittelachsen der Sacklöcher die Ecken eines gedachten Rechtecks bevorzugt eines Quadrates begrenzen. Be einer Viereranordnung hat bevorzugt jedes der Sacklöcher ein entlang der Längserstreckung des Strömungskanals benachbartes Sackloch sowie ein senkrecht dazu benachbartes Sackloch. Noch weiter bevorzugt ist es nun, wenn jedes der Sacklöcher mit dem jeweils in der Längserstreckung des Strömungskanals sowie dem senkrecht dazu benachbarten Sackloch flüssigkeitsleitend verbunden ist.

Um die Verdampfungsleistung weiter zu optimieren hat es sich als vorteilhaft herausgestellt, die Heizeinrichtung nicht seitlich versetzt zur Längsmittelachse bzw. zum Mittelpunkt eines Sacklochgrundes anzuordnen sondern derart, dass die gedachten Verlängerungen der jeweiligen Sacklochlängsmittelachse die Heizeinrichtung schneiden. Bevorzugt umfasst die Heizeinrichtung mindestens einen im Verdampferkörper angeordneten Kanal, in welchem eine Widerstandsheizung entsprechend vorheriger Definition angeordnet ist.

Die Erfindung führt auch auf eine Dekontaminationsanordnung, umfassend einen zu dekontaminierenden Raum, insbesondere einen Isolator und/oder eine Schleuse und eine nach dem Konzept der Erfindung ausgebildete Vorrichtung (Blitzverdampfer) zum Erzeugen von Dekontaminationsmitteldampf. Der Raum ist dampfleitend mit dem Auslass des Strömungskanals des Blitzverdampfers verbunden. Bevorzugt wird als Trägermedium Luft, insbesondere Umgebungsluft angesaugt und über dem Trägermedium-Einlass in den Strömungskanal eingespeist.

Die Erfindung führt auch auf ein Verfahren zum Betreiben einer nach dem Konzept der Erfindung ausgebildeten Vorrichtung zum Erzeugen von Dekontaminationsmitteldampf, wobei über den mindestens einen Zuführkanal in mindestens eines der Sacklöcher Dekontaminationsmittelflüssigkeit, insbesondere Wasserstoffperoxid zugeführt, bevorzugt eingetropft wird. Erfindungsgemäß ist nun vorgesehen, dass zumindest ein Teil einer in eines der Sacklöcher zugeführte Dekontaminationsmittelflüssigkeit über die flüssigkeitsleitende Verbindung in mindestens ein anderes der (mit diesem Sackloch flüssigkeitsleitend verbundenes) Sackloch fließt und dort zu Dekontaminationsmitteldampf verdampft wird. Aus diesem Verfahren resultiert eine erhöhte Betriebssicherheit, da auch für den Ausfall eines Zuführkanals eine größere, insbesondere die gesamte Verdampferfläche weiterhin zur Verfügung steht.

In Weiterbildung der Erfindung ist mit Vorteil vorgesehen, dass auch ein Teil der in Richtung eines benachbarten Sackloches über die flüssigkeitsleitende Verbindung fließenden Dekontaminationsmittelflüssigkeit bereits in der flüssigkeitsleitenden Verbindung verdampft wird, bevorzugt derart, dass der entstehende Dekontaminationsmitteldampf aufgeteilt auf die unmittelbar verbundenen Sacklöcher nach oben in den Strömungskanal einströmen kann. Zusätzlich oder alternativ ist vorgesehen, dass die Dekontaminationsmittelflüssigkeit über ein unmittelbar angeschlossenes bzw. flüssigkeitsleidends verbundenes Sackloch in mindestens noch ein weiteres, wiederum mit dem benachbarten Sackloch flüssigkeitsleitend verbundenes Sackloch einströmen kann.

Ganz besonders bevorzugt ist es, wenn der Volumenstrom, d.h. die Menge der pro Zeiteinheit die Gesamtzahl der Sacklöcher zugeführte Dekontaminationsmittelflüssigkeit konstant gehalten wird, insbesondere unabhängig von der Anzahl der aktuelle zur Verfügung stehenden Zuführkanäle. Dies kann dadurch realisiert werden, dass, falls einer der Zuführkanäle ausfällt sich bei konstantem Volumenstrom automatisch die Fließgeschwindigkeit in den anderen Zuführkanälen erhöht. Dadurch, dass die Sacklöcher flüssigkeitsleitend mit Abstand zum Strömungskanal miteinander verbunden sind kann sich die Dekontaminationsmittelflüssigkeit optimal verteilen und es steht dadurch eine große Verdampferfläche zur Verfügung, so dass der Betrieb des Blitzverdampfers nicht negativ beeinflusst wird und die Dekontaminationszeit nicht erhöht werden muss. Das Konstanthalten des Volumenstroms kann beispielsweise über eine Volumenstromregelung realisiert werden, wozu bevorzugt in einem Zuführstrang für Dekontaminationsmittel ein entsprechender Durchflussmesser angeordnet ist und eine Pumpe über signalleitend mit dem Durchflussmesser verbundene Steuermittel so angesteuert wird, dass der Volumenstrom zumindest näherungsweise konstant gehalten wird.

Weitere Vorteile, Merkmale und Einzelheiten ergeben sich aus der nachfolgenden Beschreibung bevorzugter Ausführungsbeispiele sowie anhand der Zeichnungen.

Diese zeigen in:
- Fig. 1:: eine Längsschnittansicht durch einen einteiligen Verdampferkörper eines Blitzverdampfers in einer vertikal verlaufenden Schnittebene,
- Fig. 2:: eine Schnittansicht durch den Verdampferkörper gemäß Fig. 1 in einer waagerechten Schnittebene,
- Fig. 3:: eine Draufsicht auf den Verdampferkörper gemäß den Fig. 1 und 2, und
- Fig. 4:: eine Schnittansicht durch den Verdampferkörper entlang einer im Wesentlichen U-förmig verlaufenden Schnittlinie.

In den Figuren sind gleiche Elemente und Elemente mit der gleichen Funktion mit den gleichen Bezugszeichen gekennzeichnet.

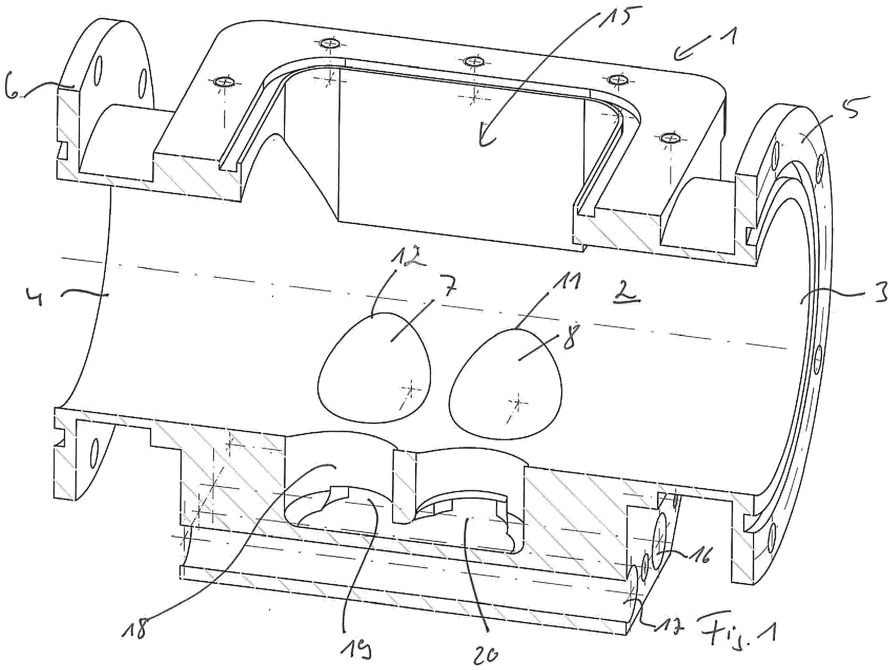

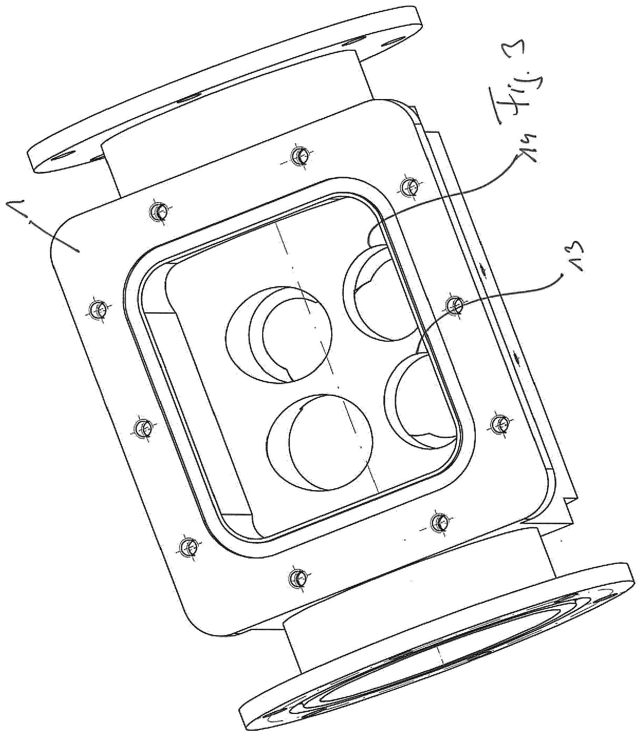

In Fig. 1 bis Fig. 4 ist ein Verdampferkörper 1 aus Vollmaterial, hier Edelstahl gezeigt. Der Verdampferkörper 1 bildet den Kernbestandteil eines ansonsten nicht näher dargestellten und beispielsweise in der EP 2 448 602 B1 beschriebenen Blitzverdampfers sowie einer dort in den Fig. 10 und 11 gezeigten (Dekontaminations-)Anordnung.

Im Verdampferkörper 1 ist ein Strömungskanal 2 ausgebildet, der einen Trägermedium-Einlass 3 mit einem Auslass 4 gasleitend verbindet. Dem Trägermedium-Einlass 3 sowie dem Auslass 4 ist jeweils ein Flansch 5, 6 zugeordnet, mit dem der Verdampferkörper 1 an entsprechende Leitungen oder im Falle des Auslasses 4 bei Bedarf direkt an einem zu dekontaminierenden Raum angeschlossen bzw. angeflanscht werden kann. Über den Trägermedium-Einlass 3 wird ein Trägermedium, insbesondere Luft zugeführt, welches dann den im Verdampferkörper 1 entstehenden Dekontaminationsmitteldampf in den zu dekontaminierenden Raum mitnimmt.

Zur eigentlichen Dekontaminationsmitteldampferzeugung sind mehrere, im vorliegenden Beispiel insgesamt vier Sacklöcher 7, 8, 9, 10 vorgesehen, die durch Bohren hergestellt sind. Ein oberer Sacklochrand 11, 12, 13, 14 befindet sich in einem gekrümmten Bereich des zylindrisch konturierten Strömungskanals unterhalb einer oberen Öffnung 15, die bei einem fertig montierten Blitzverdampfer verschlossen ist und die durchragt ist von nicht dargestellten, insbesondere von Injektionsnadeln gebildeten Zuführkanälen, die den Strömungskanal 2 senkrecht zur seiner Längserstreckung durchsetzen und jeweils in einem der Sacklöcher 7, 8, 9, 10 ausmünden. Wie im allgemeinen Beschreibungsteil erläutert, ist es nicht zwingend notwendig, jedoch bevorzugt, jedem der Sacklöcher 7, 8, 9, 10 einen eigenen Zuführkanal zuzuordnen, da die Dekontaminationsmittelflüssigkeit, wie später noch erläutert werden wird, mit Abstand zum Strömungskanal 2 verteilt werden kann/wird.

Unterhalb der Sacklöcher 7, 8, 9, 10 befinden sich parallel zum Strömungskanal 2 verlaufende Aufnahmebohrungen 16, 17, die unmittelbar unterhalb der Sacklöcher 7, 8, 9, 10 verlaufen und von den gedachten, Längsmittelachsen der Sacklöcher 7, 8, 9, 10 beschnitten werden. In den Aufnahmebohrungen 16, 17 ist beim fertig montierten Blitzverdampfer die Heizeinrichtung aufgenommen.

An den oberen Sacklochrand 11, 12, 13, 14 schließt unten jeweils ein umfangsgeschlossener Sacklochabschnitt 18 an, der jeweils benachbarte der Sacklöcher voneinander beabstandet. Dieser, eine umfangsgeschlossene Mantelfläche aufweisende Sacklochabschnitt 18 beabstandet auch den Strömungskanal 2 von diesem zugeordneten flüssigkeitsleitenden Verbindungen 19, die unterhalb des Strömungskanals 2 Sacklöcher 7, 8, 9, 10 miteinander verbinden.

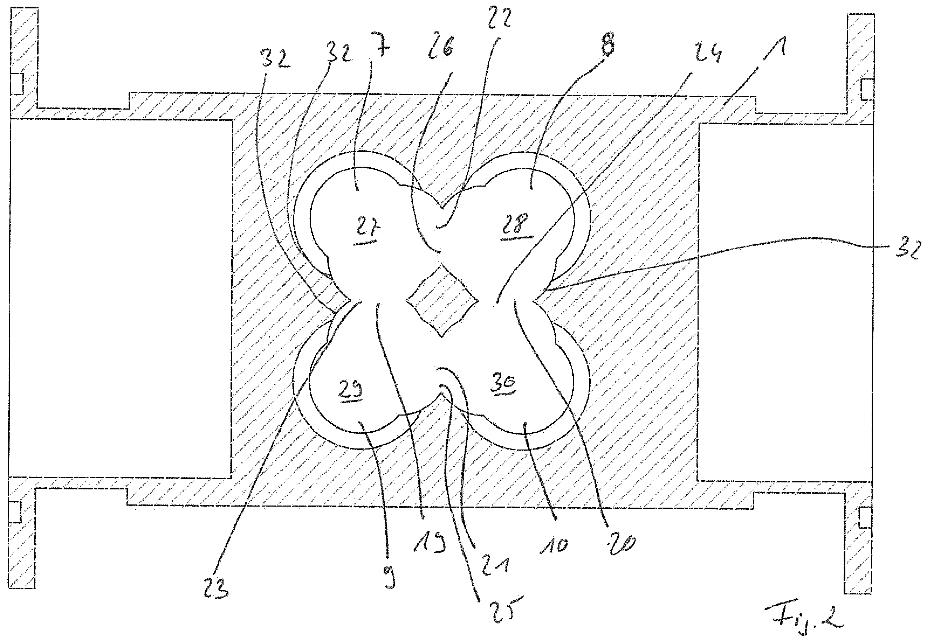

In Fig. 1 sind zwei flüssigkeitsleitende Verbindungen 19, 20 gezeigt und in Fig. 2 zusätzlich die weiteren flüssigkeitsleitenden Verbindungen 21, 22. Insbesondere aus Fig. 2 ist zu erkennen, dass die Sacklöcher 7, 8, 9, 10, genauer deren nicht eingezeichnete Längsmittelachsen in Rechteckform angeordnet sind bzw. die Ecken eines gedachten Rechteckes, hier eines gedachten Quadrates begrenzen, wobei jedes der Sacklöcher 7, 8, 9, 10 mit zwei weiteren der Sacklöcher über jeweils eine flüssigkeitsleitende Verbindung flüssigkeitsleitend verbunden ist.

Zu erkennen ist, dass die flüssigkeitsleitenden Verbindungen 19, 20, 21, 22 (vgl. insbesondere die Zusammenschau von Fig. 1 und Fig. 2) jeweils als umfangsgeschlossener Verbindungskanal 1, d.h. als eine Art Verbindungstunnel ausgebildet sind. Die flüssigkeitsleitenden Verbindungen 19, 20, 21, 22 bzw. Verbindungskanäle 1 weisen eine (tiefste) Bodenfläche 23, 24, 25, 26 auf, die die tiefsten Bereiche 27, 28, 29, 30 der Sacklöcher 7, 8, 9, 10, d.h. die Sacklochgründe in einer gemeinsamen Ebene miteinander verbindet.

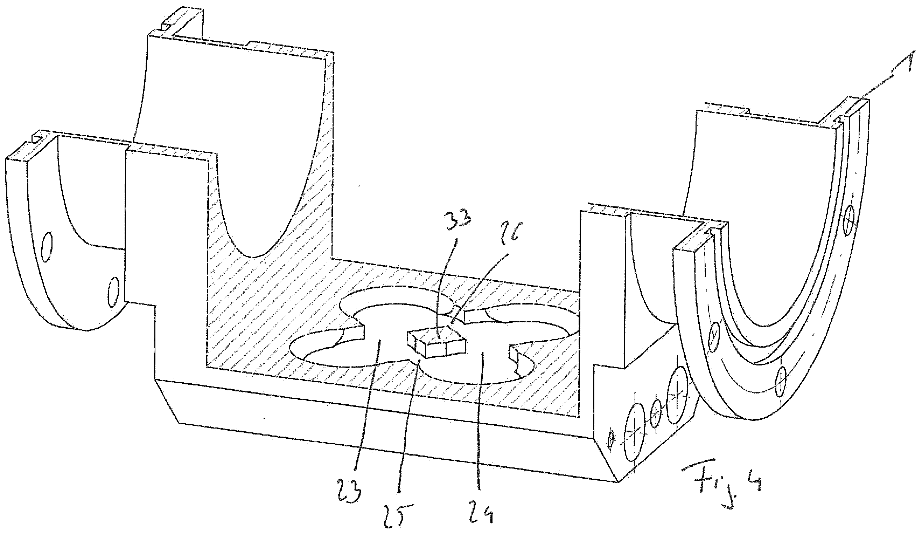

In Fig. 4 ist zu erkennen, wie die flüssigkeitsleitenden Verbindungen 19, 20, 21, 22 bzw. die Verbindungskanäle 1 hergestellt sind, nämlich durch radiales, d.h. seitliches Auffräsen, ausgehend von einer ursprünglichen Sacklochbohrung. Zu erkennen sind in Fig. 4 und Fig. 2 die entsprechenden Fräskonturen 32.

Insbesondere den Fig. 2 und 4 ist zu entnehmen, dass die im Rechteck angeordneten Sacklöcher in einem unteren Bereich, d.h. auf Höhe der flüssigkeitsleitenden Verbindungen 19, 20, 21, 22 voneinander getrennt sind über eine mittlere Säule 33, die die Verbindung herstellt zwischen dem Verdampferkörperbereich seitlich der umfangsgeschlossenen Sacklochabschnitte 18 und dem Verdampferkörperbereich unterhalb der tiefsten Bereiche 27, 28, 29, 30 der Sacklöcher 7, 8, 9, 10. Bei einer alternativen Ausführungsform kann auf eine mittlere Säule 33 verzichtet werden, indem das die Säule bildende Material, insbesondere durch seitliches Fräsen, ggf. bei engerer beieinander-Anordnung der Sacklöcher entfernt wird. Dann wird ein gemeinsamer, unterbrochener Verbindungsraum als flüssigkeitsleitende Verbindung zwischen sämtlichen Sacklöchern hergestellt. Auch eine solche flüssigkeitsleitende Verbindung zeichnet sich durch einen Deckenbereich radial benachbart zu den Sacklöchern aus.

Die in eines der Sacklöcher 7, 8, 9, 10 über einen Zuführkanal eingeleitete, insbesondere eingetropfte Dekontaminationsmittelflüssigkeit kann über die flüssigkeitsleitenden Verbindungen 19, 20, 21, 22 verteilen, so dass die Nutzung der gesamten Verdampferfläche möglich ist, auch wenn beabsichtigt oder unbeabsichtigt eines der Sacklöcher 7, 8, 9, 10 nicht von oben, unmittelbar durch einen Zuführkanal mit Dekontaminationsmittelflüssigkeit versorgt wird.

Die Ausbildung der flüssigkeitsleitenden Verbindungen 19, 20, 21, 22 jeweils als umfangsgeschlossener Verbindungskanal bedeutet, dass diese gegenüber der unteren Bodenfläche 23, 24, 25, 26 eine geschlossene Deckenfläche aufweisen sowie Deckenfläche mit der Bodenfläche verbindende, voneinander beabstandete Seitenwandflächen.

### Bezugszeichen

- 1: Verdampferkörper
- 2: Strömungskanal
- 3: Trägermedium-Einlass
- 4: Auslass
- 5: Flansch
- 6: Flansch
- 7: Sackloch
- 8: Sackloch
- 9: Sackloch
- 10: Sackloch
- 11: oberer Sacklochrand
- 12: oberer Sacklochrand
- 13: oberer Sacklochrand
- 14: oberer Sacklochrand
- 15: obere Öffnung
- 16: Aufnahmebohrung
- 17: Aufnahmebohrung
- 18: Sacklochabschnitt
- 19: flüssigkeitsleitende Verbindung
- 20: flüssigkeitsleitende Verbindung
- 21: flüssigkeitsleitende Verbindung
- 22: flüssgkeitsleitende Verbindung
- 23: Bodenfläche
- 24: Bodenfläche
- 25: Bodenfläche
- 26: Bodenfläche
- 27: tiefster Bereich
- 28: tiefster Bereich
- 29: tiefster Bereich
- 30: tiefster Bereich
- 32: Fräskanten
- 33: Säule

## Patentansprüche

1. Vorrichtung zum Erzeugen von Dekontaminationsmitteldampf, insbesondere Wasserstoffperoxiddampf, umfassend einen ein- oder mehrteiligen Verdampferkörper (1), eine Heizeinrichtung zum Erhitzen des Verdampferkörpers (1) sowie mindestens einen Zuführkanal, bevorzugt mehrere Zuführkanäle, zum Zuführen von zu verdampfender Dekontaminationsmittelflüssigkeit, insbesondere Wasserstoffperoxid, zu mindestens einem von mehreren in dem Verdampferkörper (1) angeordneten Sacklöchern (7, 8, 9, 10), und einem oberhalb von oberen Sacklochrändern (11, 12, 13, 14) der Sacklöcher (7, 8, 9, 10) angeordneten und einen Trägermedium-Einlass (3) mit einem Auslass (4) gasleitend verbindenden Strömungskanal (2) für ein gasförmiges Trägermedium, insbesondere Luft, zum Abführen des Dekontaminationsmitteldampfes in einer Strömungsrichtung des Trägermediums durch den Auslass (4),
**dadurch gekennzeichnet,**
**dass** mindestens zwei der Sacklöcher (7, 8, 9, 10) bevorzugt sämtliche Sacklöcher (7, 8, 9, 10), mit Abstand zu ihrem jeweiligen oberen Sacklochrand (11, 12, 13, 14) unterhalb eines umfangsgeschlossenen Abschnittes jedes Sacklochs (7, 8, 9, 10) flüssigkeitsleitend miteinander verbunden sind.

2. Vorrichtung nach Anspruch 1,
**dadurch gekennzeichnet,**
**dass** die flüssigkeitsleitende Verbindung (19, 20, 21, 22) derart ausgebildet ist, dass diese die tiefsten Bereiche (27, 28, 29, 30) der flüssigkeitsleitend miteinander verbundenen Sacklöcher (7, 8, 9, 10) in einer die tiefsten Bereiche (27, 28, 29, 30) aufnehmenden Verbindungsebene miteinander verbindet.

3. Vorrichtung nach einem der Ansprüche 1 oder 2,
**dadurch gekennzeichnet,**
**dass** die flüssigkeitsleitende Verbindung (19, 20, 21, 22) einen umfangsgeschlossenen Verbindungskanal im Verdampferkörper (1) umfasst, bevorzugt jeweils einen solchen Verbindungskanal zwischen zwei miteinander flüssigkeitsleitend verbundenen Sacklöchern (7, 8, 9, 10), oder einen unterbrechungsfreien Raum, der über die Sacklöcher (7 , 8, 9, 10) mit dem Strömungskanal (2) verbunden ist.

4. Vorrichtung nach einem der vorhergehenden Ansprüche,
**dadurch gekennzeichnet,**
**dass** eine Grundfläche der flüssigkeitsleitenden Verbindung (19, 20, 21, 22), bevorzugt des mindestens einen Verbindungskanals als mit der Heizeinrichtung beheizbare Verdampferfläche ausgebildet ist, so dass auf dieser erzeugter Dekontaminationsmitteldampf über die mittels der flüssigkeitsleitenden Verbindung (19, 20, 21, 22) miteinander verbundenen Sacklöcher (7, 8, 9, 10) in den Strömungskanal (2) aufsteigen kann.

5. Vorrichtung nach einem der vorhergehenden Ansprüche,
**dadurch gekennzeichnet,**
**dass** die flüssigkeitsleitende Verbindung (19, 20, 21, 22) bevorzugt durch Fräsen in radialer Richtung bezogen auf Längsmittelachsen der Sacklöcher (7, 8, 9, 10), in Vollmaterial, bevorzugt Edelstahl, hergestellt ist.

6. Vorrichtung nach einem der vorhergehenden Ansprüche,
**dadurch gekennzeichnet,**
**dass** die Sacklochränder (11, 12, 13, 14) der flüssigkeitsleitend miteinander verbundenen Sacklöcher (7, 8, 9, 10), bevorzugt sämtlicher Sacklöcher (7, 8, 9, 10) in gemeinsamer Ebene angeordnet sind.

7. Vorrichtung nach einem der Ansprüche 1 bis 5,
**dadurch gekennzeichnet,**
**dass** die Sacklochränder (11, 12, 13, 14) in einem gekrümmten, bevorzugt zylindrischen, Mantelflächenabschnitt des Strömungskanals (2) angeordnet sind.

8. Vorrichtung nach einem der vorhergehenden Ansprüche,
**dadurch gekennzeichnet,**
**dass** mindestens zwei der flüssigkeitsleitend miteinander verbundenen Sacklöcher (7, 8, 9, 10) in Richtung der Strömungsrichtung des Trägermediums voneinander beabstandet angeordnet sind und/oder dass mindestens zwei der flüssigkeitsleitend miteinander verbundenen Sacklöcher (7, 8, 9,10) senkrecht zur Strömungsrichtung des Trägermediums voneinander beabstandet angeordnet sind.

9. Vorrichtung nach einem der vorhergehenden Ansprüche,
**dadurch gekennzeichnet,**
**dass** die Heizeinrichtung unmittelbar unterhalb jedes Sacklochgrundes in der gedachten Verlängerung der jeweiligen Sacklochlängsmittelachse im Verdampferkörper (1) angeordnet ist.

10. Anordnung, umfassend einen zu dekontaminierenden Raum, insbesondere einen Isolator und/oder eine Schleuse, und eine Vorrichtung nach einem der vorhergehenden Ansprüche, mit welcher der Raum mit Dekontaminationsmitteldampf beaufschlagbar ist.

11. Verfahren zum Betreiben einer Vorrichtung zum Erzeugen von Dekontaminationsmitteldampf, insbesondere Wasserstoffperoxiddampf, nach einem der Ansprüche 1 bis 9, wobei über den mindestens einen Zuführkanal in mindestens eines der Sacklöcher (7, 8, 9, 10) Dekontaminationsmittelflüssigkeit, insbesondere Wasserstoffperoxid, zugeführt, bevorzugt eingetropft, wird,
**dadurch gekennzeichnet,**
**dass** in eines der Sacklöcher (7, 8, 9, 10) zugeführte Dekontaminationsmittelflüssigkeit über die flüssigkeitsleitende Verbindung (19, 20, 21, 22) in mindestens ein anderes der Sacklöcher (7, 8, 9, 10) fließt und dort zu Dekontaminationsflüssigkeitsdampf verdampft wird.

12. Verfahren nach Anspruch 11,
**dadurch gekennzeichnet,**
**dass** ein Teil der durch die flüssigkeitsleitende Verbindung (19, 20, 21, 22) fließende Dekontaminationsmittelflüssigkeit in der flüssigkeitsleitenden Verbindung (19, 20, 21, 22) verdampft und/oder dass ein Teil der durch die flüssigkeitsleitende Verbindung (19, 20, 21, 22) fließende Dekontaminationsmittelflüssigkeit über das andere Sackloch (7, 8, 9, 10) und eine weitere flüssigkeitsleitende Verbindung (19, 20, 21, 22) zu einem noch weiteren Sackloch (7, 8, 9, 10) fließt.

13. Verfahren nach einem der Ansprüche 11 oder 12,
**dadurch gekennzeichnet,**
**dass** der dem Verdampferkörper (1) zugeführte Gesamtvolumenstrom der Dekontaminationsmittelflüssigkeit konstant gehalten wird.

## Claims

1. A device for generating decontaminant fumes, in particular hydrogen peroxide fumes, comprising an evaporator body (1) in one or several pieces, a heating device for heating the evaporator body (1) as well as at least one supply duct, preferably several supply ducts, for supplying decontaminant liquid to be evaporated, in particular hydrogen peroxide, to at least one of several blind bores (7, 8, 9, 10) arranged in the evaporator body (1), and a flow duct (2) for a gaseous carrier medium, in particular air, for discharging the decontaminant fumes through an outlet (4) in a flow direction of the carrier medium, said flow duct (2) being arranged above upper blind bore edges (11, 12, 13, 14) of the blind bores (7, 8, 9, 10) and connecting a carrier-medium inlet (3) to the outlet (4) in order to conduct gas,
**characterized in that**
at least two of the blind bores (7, 8, 9, 10), preferably all blind bores (7, 8, 9, 10), are connected to each other in order to carry liquid and below a section, having a fully closed perimeter, of each blind bore (7, 8, 9, 10) and at a distance to the respective upper blind bore edge (11, 12, 13, 14).

2. The device according to claim 1,
**characterized in that**
the connection (19, 20, 21, 22) carrying liquid is realized such that they connect the deepest areas (27, 28, 29, 30) of the blind bores (7, 8, 9, 10), which are connected to each other in order to carry liquid, to a connecting plane receiving the deepest areas (27, 28, 29, 30) to each other.

3. The device according to claim 1 or 2,
**characterized in that**
the connection (19, 20, 21, 22) carrying liquid comprises a connecting duct, which has a fully closed perimeter, in the evaporator body (1), preferably such a connecting duct between two blind bores (7, 8, 9, 10) in each instance connected to each other in order to carry liquid, or comprises an uninterrupted space, which is connected to the flow duct (2) via the blind bores (7, 8, 9, 10).

4. The device according to any one of the preceding claims,
**characterized in that**
a basic surface of the connection (19, 20, 21, 22) carrying liquid, preferably of the at least one connecting duct, is realized as an evaporation surface, which can be heated by means of the heating device, so that decontaminant fumes generated on said evaporation surface can rise into the flow duct (2) via the blind bores (7, 8, 9, 10) connected to each other by means of the connection (19, 20, 21, 22) carrying liquid.

5. The device according to any one of the preceding claims,
**characterized in that**
the connection (19, 20, 21, 22) carrying liquid is preferably formed by milling in a radial direction with respect to longitudinal middle axes of the blind bores (7, 8, 9, 10) in solid material, preferably stainless steel.

6. The device according to any one of the preceding claims,
**characterized in that**
the blind bore edges (11, 12, 13, 14) of the blind bores (7, 8, 9, 10) connected to each in order to carry liquid, preferably of all blind bores (7, 8, 9, 10), are arranged in a shared plane

7. The device according to any one of the claims 1 to 5,
**characterized in that**
the blind bore edges (11, 12, 13, 14) are arranged in a curved, preferably cylindrical, jacket surface section of the flow duct (2).

8. The device according to any one of the preceding claims,
**characterized in that**
at least two of the blind bores (7, 8, 9, 10) connected to each other in order to carry liquid are arranged at a distance to each other in the direction of the flow direction of the carrier medium and/or that at least two of the blind bores (7, 8, 9, 10) connected to each other in order to carry liquid are arranged at a distance to each other perpendicular to the flow direction of the carrier medium.

9. The device according to any one of the preceding claims,
**characterized in that**
the heating device is arranged directly below each blind bore bottom in the imagined extension of the respective longitudinal central axis of the blind bore in the evaporator body (1).

10. An arrangement, comprising a room to be decontaminated, in particular an isolator and/or an airlock, and a device according to any one of the preceding claims, by means of which the room can be pressurized with decontaminant fumes.

11. A method for operating a device for generating decontaminant fumes, in particular hydrogen peroxide fumes, according to any one of the claims 1 to 9, decontaminant liquid, in particular hydrogen peroxide, being supplied, preferably drop by drop, into at least one of the blind bores (7, 8, 9, 10) via the at least one supply duct,
**characterized in that**
decontaminant liquid supplied in one of the blind bores (7, 8, 9, 10) flows in at least one other blind bore (7, 8, 9, 10) via the connection (19, 20, 21, 22) carrying liquid and evaporates there to decontaminant fumes.

12. The method according to claim 11,
**characterized in that**
a part of the decontaminant liquid flowing through the connection (19, 20, 21, 22) carrying liquid evaporates in the connection (19, 20, 21, 22) carrying liquid and/or in that a part of the decontaminant liquid flowing through the connection (19, 20, 21, 22) carrying liquid flows to yet another blind bore (7, 8, 9, 10) via the other blind bore (7, 8, 9, 10) and another connection (19, 20, 21, 22) carrying liquid.

13. The method according to claim 11 or 12,
**characterized in that**
the entire volume flow of the decontaminant liquid supplied to the evaporator body (1) remains constant.

## Revendications

1. Dispositif pour générer du vapeur de décontaminant, en particulier du peroxyde d'hydrogène, comprenant un corps (1) d'évaporation en une ou plusieurs pièces, un dispositif de chauffage pour chauffer le corps (1) d'évaporation ainsi qu'au moins un conduit d'alimentation, de préférence plusieurs conduits d'alimentation, pour alimenter du liquide de décontaminant, en particulier du peroxyde d'hydrogène, à au moins un de plusieurs trous (7, 8, 9, 10) borgnes disposés dans le corps (1) d'évaporation, et un conduit (2) d'écoulement pour un médium porteur gazeux, en particulier l'air, pour décharger le vapeur de décontaminant par une sortie (4) dans un sens d'écoulement du médium porteur, ledit conduit (2) d'écoulement étant disposé au-dessus des bords (11, 12, 13, 14) supérieurs de trou borgne des trous (7, 8, 9, 10) borgnes et reliant une entrée (3) de médium porteur à la sortie (4) de manière à guider du liquide,
**caractérisé en ce qu'**
au moins deux des trous (7, 8, 9, 10) borgnes, de préférence tous les trous (7, 8, 9, 10) borgnes, sont reliés les uns aux autres de manière à guider du liquide et au-dessous d'une section ayant une périphérie fermée de chaque trou (7, 8, 9, 10) borgne et ayant une distance au bord (11, 12, 13, 14) supérieur correspondant de trou borgne.

2. Dispositif selon la revendication 1,
**caractérisé en ce que**
la connexion (19, 20, 21, 22) portant du liquide est réalisée de telle manière qu'elle relie les zones (27, 28, 29, 30) les plus profondes des trous (7, 8, 9, 10) borgnes, qui sont reliés les uns aux autres de manière à guider du liquide, les unes aux autres dans une plane de connexion recevant les zones (27, 28, 29, 30) les plus profondes.

3. Dispositif selon la revendication 1 ou 2,
**caractérisé en ce que**
la connexion (19, 20, 21, 22) portant du liquide comprend un conduit de connexion, qui a une périphérie fermée, dans le corps (1) d'évaporation, de préférence un tel conduit de connexion toujours entre deux trous (7, 8, 9, 10) borgnes reliés les uns aux autres de manière à guider du liquide, ou une chambre ininterrompue qui est reliée au conduit (2) d'écoulement par le trous (7, 8, 9, 10) borgnes.

4. Dispositif selon l'une quelconque des revendications précédentes,
**caractérisé en ce qu'**
une surface de base de la connexion (19, 20, 21, 22) portant du liquide, de préférence d'au moins un conduit de connexion, est réalisée de manière à avoir une surface d'évaporation, qui peut être échauffée au moyen du dispositif de chauffage, afin que du vapeur de décontaminant généré sur ladite surface d'évaporation peut élever dans le conduit (2) d'écoulement par les trous (7, 8, 9, 10) borgnes, qui sont reliés les uns aux autres au moyens de la connexion (19, 20, 21, 22) portant du liquide.

5. Dispositif selon l'une quelconque des revendications précédentes,
**caractérisé en ce que**
la connexion (19, 20, 21, 22) portant du liquide est produite préférablement par être fraisée en matériau plein, de préférence en acier inoxydable, dans le sens radial en relation aux axes centraux longitudinaux des trous (7, 8, 9, 10) borgnes.

6. Dispositif selon l'une quelconque des revendications précédentes,
**caractérisé en ce que**
les bords (11, 12, 13, 14) de trou borgne des trous (7, 8, 9, 10) borgnes reliés les uns aux autres de manière à guider du liquide, de préférence de tous les trous (7, 8, 9, 10) borgnes, sont disposés dans une plane commune.

7. Dispositif selon l'une quelconque des revendications précédentes,
**caractérisé en ce que**
les bords (11, 12, 13, 14) de trou borgne sont disposés dans une partie de surface d'enveloppe courbée, de préférence cylindrique, du conduit (2) d'écoulement.

8. Dispositif selon l'une quelconque des revendications précédentes,
**caractérisé en ce qu'**
au moins deux des trous (7, 8, 9, 10) borgnes reliés les uns aux autres de manière à guider du liquide sont disposés à être espacés les uns des autres dans le sens d'écoulement du médium porteur et/ou **en ce qu'**au moins deux des trous (7, 8, 9, 10) borgnes reliés les uns aux autres de manière à guider du liquide sont disposés à être espacés les uns des autres perpendiculairement au sens d'écoulement du médium porteur.

9. Dispositif selon l'une quelconque des revendications précédentes,
**caractérisé en ce que**
le dispositif de chauffage est disposé directement au-dessous de chaque fond de trou borgne dans le prolongement imaginaire de l'axe central longitudinal correspondant de trou borgne dans le corps (1) d'évaporation.

10. Ensemble, comprenant une chambre à être décontaminée, en particulier un isolateur et/ou un sas, et un dispositif selon l'une quelconque des revendications précédentes au moyen duquel la chambre peut être pressurisée avec du vapeur de décontaminant.

11. Procédé pour opérer un dispositif pour générer du vapeur de décontaminant, en particulier du peroxyde d'hydrogène, selon l'une quelconque des revendications 1 à 9, du liquide de décontaminant, en particulier du peroxyde d'hydrogène, étant alimenté, de préférence goutte-à-goutte, dans au moins un des trous (7, 8, 9, 10) borgnes au moyen d'au moins un conduit d'alimentation,
**caractérisé en ce que**
du liquide de décontaminant alimenté dans un des trous (7, 8, 9, 10) borgnes s'écoule dans au moins un autre des trous (7, 8, 9, 10) borgnes au moyen de la connexion (19, 20, 21, 22) portant du liquide et est y évaporé au vapeur de liquide de décontaminant.

12. Procédé selon la revendication 11,
**caractérisé en ce qu'**
un part du liquide de décontaminant écoulant par la connexion (19, 20, 21, 22) portant du liquide s'évapore dans la connexion (19, 20, 21, 22) portant du liquide et/ou **en ce qu'**un part du liquide de décontaminant écoulant par la connexion (19, 20, 21, 22) portant du liquide s'écoule vers un outre trou (7, 8, 9, 10) borgne au moyen de l'autre trou (7, 8, 9, 10) borgne et d'une autre connexion (19, 20, 21, 22) portant du liquide.

13. Procédé selon la revendication 11 ou 12,
**caractérisé en ce que**
le débit volumétrique global du liquide de décontaminant alimenté au corps (1) d'évaporation est maintenu constant.
